# EUROPEAN PATENT APPLICATION

(11) **EP 4 841 572 A1**
(43) Date of publication of application: **18.11.2026**
(21) Application number: 25176754.7
(22) Date of filing: 15.05.2025
(51) Int. Cl.: G06T 15/06, G16H 50/00

(54) **RENDERING SCALAR AND MULTI-SPECTRAL 3D MEDICAL IMAGE DATA**

(71) Applicant: Siemens Healthineers AG, 91301 Forchheim (DE)
(72) Inventor: Bohn, Lars, 91077 Neunkirchen am Brand (DE); Dingeldey, Felix, 91056 Erlangen (DE)
(74) Representative: Siemens Healthineers Patent Attorneys

(57) **Abstract**

This invention relates to a computer-implemented method for rendering medical visualizations derived from 3D volume data obtained via a 3D imaging device (ID). The method employs a rendering algorithm to visualize reconstructed volume data, involving ray generation, sampling, surface intersection detection, and shading. During surface intersection detection, the method calculates intersection points for transition interval boundaries, applies detection functions, and combines surface colors for final color computation. In shading, it utilizes sampling values and applies a shading function influenced by transition interval properties, as well as assessing material properties and BxDF parameters. The final step involves displaying the rendered visualization.

## Description

High quality of medical imaging of 3D data is important for improving diagnosis, treatment and treatment planning based on image data, and patient outcomes. In particular, improving quality of 3D image rendering helps visualizing the exact size, shape, and location of organs or lesions for instance for planning minimally invasive procedures, spotting abnormalities more easily (e.g., tumors, fractures, aneurysms), distinguishing between overlapping tissues or between healthy structures and lesions, as well as for better monitoring of disease progression or healing over time by comparing volumetric data, which is often more sensitive to subtle changes than flat images.

The field of medical imaging and visualization involves capturing detailed - optionally multidimensional - information from within the human body using 3D imaging devices such as CT (Computed Tomography), MRI (Magnetic Resonance Imaging), and PET (Positron Emission Tomography) scanners. The data output from these devices is typically volumetric in nature, representing complex 3D structures or volumes of tissues, organs, and other anatomical features. Typically, such an imaging device is used to acquire a 3D dataset of raw data. Depending on the type of imaging modality, these raw data are subject to further processing, in particular for applying rendering algorithms for visualizing the volumetric data. In state of the art, it is known to use a plurality of different rendering algorithms on the reconstructed volumetric data.

Rendering technology plays a crucial role in this domain, providing the ability to convert raw volume data, acquired by one of the above-mentioned 3D imaging devices (also mentioned: modalities), into comprehensible visual representations which can be analyzed by medical professionals. These visualizations help in diagnostics, treatment planning, and research by allowing the detailed examination of anatomical structures in three dimensions, complete with the ability to interactively explore different perspectives and layers within the data.

Key components of medical visualization systems include rendering algorithms, which process raw or reconstructed volumetric data to create images suitable for display. These algorithms often incorporate advanced techniques like ray tracing to simulate realistic lighting and shadows, enhancing the detail and depth of the final image. Surface detection and shading models might be integral to this process, contributing to the accuracy and realism of the visualization by modeling how light interacts with surfaces and materials based on their properties.

In the context of medical visualization, developments are aimed at improving the fidelity and efficiency of rendering processes, enabling more accurate and intuitive representations of complex biological structures. This includes the optimization of reconstruction methods to handle large datasets and the development of algorithms that enhance the clarity and usefulness of the visualizations while reducing computational overhead. Furthermore, combining visualization techniques with user interface software enhances interactivity, empowering physicians to manipulate and interrogate the data with ease, potentially leading to faster and more accurate diagnoses.

Medical 3D imaging data can be visualized for instance with the Shaded Surface Display (SSD) Rendering Algorithm or more recently with global illumination variants, such as Path-traced SSD. These techniques use the result of an image reconstruction that takes place immediately after the actual acquisition by the modality (such as CT, MRI, or PET).

The conventional SSD rendering algorithm has several limitations when processing and visualising multi-spectral and scalar data. In particular, there is a lack of specific support for multi-spectral data, which makes it difficult to analyse complex data. This leads to limited functionalities when displaying very complex scalar data, which can lead to inaccurate results or time-consuming processes. These challenges make it difficult to achieve accurate and efficient visualisations required for advanced imaging tasks, especially in the field of medical imaging.

The integration of multi-spectral imaging is increasingly prevalent, providing additional depth to the visualizations by capturing data across multiple wavelengths. This approach augments standard imaging modalities, offering richer data sets from which to derive representations, and allowing the incorporation of more detailed information (e.g., different biological markers) that can be instrumental in identifying pathologies.

With the advent of multi-spectral imaging (such as CT Dual Energy, CT Photon Counting or multi-spectral MRI), it becomes possible to t acquire data at multiple energy levels or spectral bands, allowing for enhanced tissue characterization, material decomposition, and improved diagnostic accuracy. Multi-spectral imaging may for instance refer to Dual-Energy CT (DECT), Photon-Counting CT (PCCT), and Multi-spectral MRI.

Up until now, multiple reconstruction results have only been rendered together into a single result image using simple blend operations (fusion rendering). For this, each reconstructed volume is rendered individually, and the resulting images are combined into a final image.

This significantly limits the potential of visualizing multi-spectral imaging data since only the resulting pixel value is considered. This resulting pixel color value at the final intersection position is used for compositing the result image.

In state of the art, independently of the multi-spectral case, current SSD methods use a single color for defining the surface material and appearance. This limits the information that can be conveyed, since many properties of the intersection detection are left unused.

Based on the above, it is an object of the present invention to provide an improved rendering and to improve the visualization quality of medical data and to overcome the disadvantages of the known methods in the state of the art, as discussed above. In particular, the SSD algorithm and its variants, in particular the path-traced variants, should be improved by using additional information from reconstruction. Furthermore, rendering of multi-spectral image data should be improved by the ability for considering additional information from reconstruction.

This object is achieved by a computer-implemented method for rendering medical visualizations, acquired by a 3D imaging device according to claim 1, a rendering machine and a medical image processing system according to the attached independent claims.

The solution described herein revolves around medical imaging technology, specifically related to the visualization and processing of 3D volume data acquired via imaging devices such as multi-spectral or other advanced imaging systems. The focus is on computer-implemented methods and systems for efficiently rendering medical visualizations of volume data, using various computational algorithms. These methods involve complex steps like ray generation, sampling for determining values, surface intersection detection, and shading to create a meaningful visualization that aids in medical diagnosis or analysis. The disclosure also discusses components like rendering machines and medical image processing systems that facilitate these visualizations, underlining the integration of hardware and software for enhanced medical imaging capabilities.

In the following, the invention will be described with respect to the claimed method first. Features, advantages, or alternative embodiments, mentioned with respect to the method can be assigned to the other claimed objects (e.g. the rendering machine or the medical system or to computer program or a computer program product) and vice versa. In other words, the system, apparatus, or device can be improved with features described or claimed in the context of the method and vice versa. In this case, the functional features of the method are embodied by structural units of the apparatus or device or system and vice versa, respectively. The method may refer to a software implementation and the device may refer to a hardware implementation (e.g. with a spatial physical structure) or a virtualization thereof. Generally, in computer science a software implementation and a corresponding hardware implementation (e.g. as an embedded system) are equivalent. Thus, for example, a method step for "receiving" data may be performed with a respective reception interface configured to receive data. For the sake of avoiding redundancy, although the device may also be used in the alternative embodiments described with reference to the method, these embodiments are not explicitly described again for the device. In principle, the respective device or apparatus claim is configured to carry out the claimed method.

According to a first aspect, the present invention refers to a computer-implemented method for rendering medical visualizations.

For rendering, it is known to use rendering algorithms, in particular like SSD or path-traced SSD variants. These conventional rendering algorithms are modified in that certain steps are adapted. This adaption or modification is explained below in more detail.

The method may comprise executing a (thus modified or adapted) rendering algorithm for calculating a visualization for a previously provided reconstruction result. The reconstruction result comprises a 3D volume (also: volume data), acquired by a 3D imaging device (also: image acquisition device. Generally, when executing the rendering algorithm, at least the following steps may be executed: ray generation, sampling for determining sampling values, surface intersection detection and shading.

In the step of surface intersection detection for calculating at least one surface intersection point, the method and in particular the rendering algorithm is adapted for calculating, a first surface intersection point for a lower boundary of a transition interval and calculating a second surface intersection point for an upper boundary of the transition interval and applying a detection function on the calculated first and second surface intersection point for determining the surface intersection point and/or rendering two surfaces, namely at the lower and the upper boundary and composing two surface colours of the rendered surfaces to calculate a final colour for the ray.

Alternatively or in addition, in the step of shading, the method and in particular the rendering algorithm is adapted for using the determined sampling values which have been determined during the step of sampling - in particular instead of the material colour. Alternatively or cumulatively the method and in particular the rendering algorithm is adapted for determining a surface colour by using a shading function, which is based on properties of the transition interval and/or determining material properties and/or parameters of a BxDF function, based on the properties of the transition interval.

The method may continue with displaying the rendered visualization.

Alternatively or in addition, parameters of the algorithm (like thresholds, boundary interval, selection of bidirectional distribution functions for modelling the surface light interactions of an object, the definition of masks, and/or weighting factors in the step of surface intersection detection, etc.) may be set via signals provided on the user interface.

The raw data are the data directly provided at the imaging device. The raw data are thus acquisition data and are not usable for visualization. For a scalar imaging device, the raw data are directly processed or reconstructed to provide a 3D volume, which may be used for visualization, in particular for 2D visualization. For a multi-spectral imaging device, the raw data are processed by mainly two steps: First, an intermediate result is calculated from the raw data and second, based on the intermediate result, different 3D volumes may be calculated, which are denoted as representations. The rendering algorithm may be applied on a set of representations for one and the same volume to provide a rendered visualization which may be used for display. The intermediate result is modality-specific. Alternatively or in addition, the respective intermediate result is generated in such a way that allows quick generation of different final representations.

The reconstruction result is a 3D volume which may be represented as a set of images. Further specification of a reconstruction result depends on the type of image acquisition which has been used, in particular single-spectral image acquisition or multi-spectral image acquisition. For single spectral image acquisition, the reconstruction result is one single representation, representing or comprising a scalar volume, in particular comprising a set of voxels. For multi-spectral image acquisition, the reconstruction result is a set of representations, wherein each representation is a scalar volume and wherein all representation coincide spatially.

The term "BxDF function" is to be construed as a unifying term that describes the combination of bidirectional distribution functions used for modelling the surface light interactions of an object. Functions may comprise BRDFs, BTDFs, BSSRDFs and/or BSSTDFs for modelling reflectance or transmittance of conventional and of sub-surface scattering surfaces respectively.

The composing of two surface colours may comprise selecting two different surface colours, for instance two different grey values.

In the step of shading, the step of determining material properties and/or parameters of a BxDF function may, for example, relate to determining opacity, specularity, roughness, anisotropy, refraction, and/or sub-surface scattering parameters.

A key idea of the present invention is to be seen in that the rendering algorithm does not only use one single representation of a reconstructed volume, but instead uses multiple reconstruction results. In particular, characteristics and/or values of different representations of the same volume acquired by a multi-spectral image acquisition device may be processed in the rendering algorithm.

In general, the rendering algorithm processes reconstructed data. Rendering is thus construed as a process to be executed after reconstruction or on reconstructed data. Depending on the respective technical setting, it is possible to rely on reconstructed data, stored in a storage. In this case, the 3D image acquisition and/or the reconstruction on the raw data, provided with the image acquisition have already been executed previously and the results thereof are stored in a storage. The rendering algorithm reads data from this storage. In another technical setting, the image acquisition and/or the reconstruction are executed hand in hand with or interleaved or quasi parallel to the rendering. Thus, alternatively or cumulatively, the method may comprise at least one of the following steps:
- Executing a 3D image acquisition at the 3D imaging device for providing a raw dataset; and/or
- Providing a reconstruction result with a 3D volume, reconstructed from the provided raw dataset as a representation of a scalar volume.

Alternatively or cumulatively, the rendering algorithm can utilize specific algorithms designed to enhance the display of medical imagery. In particular, the rendering algorithm may be an SSD or path-traced SSD. This improves quality of the rendering by considering global illumination.

The shaded surface display algorithm, SSD, focuses on visualizing surfaces within volumetric data by using threshold segmentation to identify and display object boundaries. In pseudocode, the SSD may be explained as: For each ray, find a surface intersection position by detecting the boundary transition of the values along the viewing ray into a transition interval defined by a lower and an upper boundary or threshold that is closest to the camera.

In particular, the SSD algorithm comprises the following steps:
1. Ray Generation
   a. Using a virtual camera, cast rays from each pixel of the camera's image plane into the virtual 3D scene in which the volumetric data is positioned in space.
   b. For each ray, determine the line segment that intersects the volume.
   c. Note: each ray represents a line of sight from the viewer. The origin and direction of the ray is defined by the camera's position, orientation, and parameters for the projection.
2. Sampling
   a. For each ray, sample values from the 3D volume at regular intervals (with the step size being defined by the application / outside the algorithm).
   b. Use an interpolation method (e.g. nearest neighbour, tri-linear, tri-cubic; also defined outside the algorithm) for reconstructing sample values, since sample positions generally do not coincide with the voxel centres.
3. Surface Intersection Detection (the output thereof is a surface intersection point)
   a. During traversal and sampling, detect the crossing of a threshold value that defines the surface.
   b. The threshold crossing is considered to denote the ray-surface intersection and an intersection point.
   c. Note: this threshold is also known as isovalue
   d. It is possible to define a lower and upper threshold. In this case, the ray-surface intersection is given as soon as the ray crosses either of the thresholds and, thus, enters the threshold interval.
4. Shading
   a. Simulate the light-surface interaction by applying an illumination model (typically Phong BRDF illumination model).
   b. Most illumination models need the surface normal, which is typically calculated from the negated, normalized gradient at the surface intersection point.
   c. Other parameters of the illumination model (e.g. light direction, surface material colour, specularity, ...) are defined by the application / outside the algorithm.

Path-Traced Shaded Surface Display (SSD) refers to a rendering technique and is a path-traced variant of the conventional SSD that enables the simulation of global illumination effects. Path-tracing is used to generate realistic shading and lighting effects on 3D surfaces, for providing photorealistic effects in medical images. Path-tracing is a Monte Carlo-based ray tracing algorithm that simulates how light interacts with surfaces by tracing multiple random paths from the camera to the scene. It models real-world lighting effects such as soft shadows, reflections, refractions, global illumination, and caustics with high accuracy.

In particular, the path-traced SSD comprises
1. Ray Generation
   a. In Monte-Carlo based path-tracing, stochastic sampling of the camera's image plane is used by casting multiple rays (often several hundreds) per pixel.
   b. Each of these rays has a randomized origin.
2. Sampling per path
   a. Same steps as in conventional SSD
3. Surface Intersection Point Detection per path
   a. Same steps as in conventional SSD
4. Shading per path
   a. Path-tracing simulates global illumination effects and indirect lighting by integrating incident light from multiple directions.
   b. At each surface intersection, a new randomized ray is generated and traversed (starting a point 1).
   c. The light contribution of the scattered ray is modulated by the illumination model to define the shading.

Sampling points might be used to estimate gradients for shading, while surface intersection points are essential for precise light reflection calculations.

Purpose of ray generation: Rays are generated from the viewpoint (camera) to traverse the volumetric dataset and determine visible surfaces.

Purpose of the step of surface intersection detection: Identify the surfaces within the volume that should be displayed based on intensity thresholds or gradients.

Purpose of shading: Apply lighting effects to enhance depth perception and realism in the rendered image. The shaded surface is displayed with varying levels of brightness and darkness, emphasizing anatomical structures.

The method may be implemented as part of an application. The application comprises a user interface for user interaction. In particular, a user may choose the rendering algorithm to be applied during runtime.

Independently of using a multi-spectral image acquisition device, and thus when using a single-spectral image acquisition device or a multi-spectral image acquisition device, the rendering algorithm, in particular, an SSD algorithm or path-traced variants thereof, may be modified by using properties of the step of surface intersection detection. Thus, not only a single colour is used for defining surface material and appearance.

The shaded surface display algorithm is adaptable for visualizing surfaces within three-dimensional volumes, emphasizing surface characteristics by incorporating shading techniques. By managing light interactions, this algorithm can highlight surface details, thus revealing beneficial medical information against complex backgrounds. SSD aims to align the user's perception with real-world surface textures and contours in the medical data, providing clarity which can support diagnostic accuracy.

Another option, the path-traced SSD, offers advanced rendering through path-tracing methodologies, which simulate the effects of light following varied paths within the volume data. Path-tracing calculates interactions of light with surfaces and materials to achieve detailed and realistic visual outcomes. This approach may enhance shading and highlighting, effectively illustrating layers and transitions within medical datasets. Using path-traced SSD might offer superior visual quality and depth perception compared to traditional methods, potentially increasing the granularity and realism of medical images for analytical use.

Utilizing these algorithms within the rendering method supports a high level of detail and precision, accommodating medical professionals' needs for informative visualizations, possibly aiding diagnostic processes.

The provided supplementary information focuses on enhancements to the rendering algorithms previously described. In particular, it discusses the extension of the Shaded Surface Display (SSD) algorithm and its path-traced variants to handle both scalar and multi-spectral data within medical imagery rendering processes. Scalar data refers to unidimensional datasets that capture single-channel information, common in traditional grayscale imaging, where nuances in light intensity across the medical image can aid diagnostics. Multi-spectral data, on the other hand, encompasses several channels of data (e.g. across different wavelengths), allowing for a significantly broader spectrum of information to be captured, akin to color data but with the channels encoding different quantities (e.g. wavelengths) depending on the imaging device.

The extension of the SSD algorithm for scalar data involves refining the rendering techniques to elevate the contrast and detail in single-channel imaging scenarios, faithfully rendering surface textures and contours useful for identifying subtle variations within anatomical structures. This improvement aims to enhance the algorithm's sensitivity to gradations in light and shade, which can assist in extrapolating meaningful insights from grayscale images that may contain nuanced diagnostic information.

Incorporating path-traced elements into this extension may increase fidelity in rendering by simulating complex light interactions across single-channel scalar datasets. This approach potentially improves the visualization of depth and material interactions, providing an immersive visual experience and possibly revealing diagnostically significant features that might remain less visible using standard rendering techniques.

When applied to multi-spectral data, the enhancement involves a strategic adjustment of both the shaded surface display and the path-traced rendering algorithms to manage and visualize multiple data channels simultaneously. Multi-spectral imaging, abundant in data, often requires advanced algorithms to differentiate and render the composite information efficiently. The extension ensures that the SSD methodology is adapted to deal with the complexity brought about by multiple data channels, leveraging the full potential of the data by allowing to provide more information and/or convey more information to the viewer.

The integration of path-tracing techniques within multi-spectral datasets offers a promising improvement in rendering quality, simulating light paths through the multi-variate data and uncovering hidden details. This capability is particularly valuable in medical imaging scenarios where diverse biological materials might present varying responses across different dimensions in the data, e.g., like different spectral ranges, thus facilitating the identification of diagnostic features that might be otherwise less discernible and contributing to comprehensive multidimensional medical analysis.

Using these enhanced algorithms with scalar and/or multi-spectral data may improve the clarity of medical visualizations and support intricate diagnostic practices by providing a new level of detail and interpretive clarity. This inventive approach in expanding the application of rendering technologies signifies a step forward in the inclusion of sophisticated data modalities within medical imaging, indicating a proactive approach to the development of diagnostic technologies that could positively affect the accuracy and efficiency of medical evaluations.

The described method includes an advanced surface intersection detection within the rendering algorithm to facilitate the precision and visibility of medical visualizations derived from volume data. In this context, the detection function incorporates several high-quality techniques that identify surface intersection points, contributing to the definition of surfaces in the visualized data.

The process may start with determining either the calculated first or second surface intersection point as the detected intersection point. This determination could be based on sampled values or properties derived from the respective positions of the intersection points. These sampled values often highlight distinctive volumetric features, assisting the detection function in selecting the surface intersection point with significant relevance.

Alternatively or cumulatively the process may involve applying a weighted average to process both the first and second surface intersection points. This average uses a weighting factor, which might be defined by various inputs: applications, signals presented on user interfaces, or properties derived from the volume data-either generally across the dataset or specific to the area around the boundaries of the transition interval. This approach offers customization and adaptability, aligning intersection detection with specific diagnostic needs and context.

Alternatively or cumulatively, the detection function may optionally determine the position within the transition interval exhibiting the largest gradient magnitude as the surface intersection point. Gradient magnitude might suggest notable changes in intensities or characteristics within the volume data, pointing to areas of medical interest or boundary definitions. This determination could reveal underlying anatomical structures or materials with enhanced visibility and resolution.

Alternatively or cumulatively, precision can be improved by identifying a position where the surface is most accurately defined, based on the highest continuity in sample values within the neighborhood. This criterion could be useful as it establishes intersections among consistent data points, enhancing imaging clarity. Similarly, focusing on the highest continuity of gradients within the neighborhood might refine the detection process by selecting areas where gradient transitions are smooth and coherent, further boosting intersection accuracy.

Integrating these advanced methods in surface intersection detection supports the rendering process, offering detailed and reliable medical visualizations beneficial for medical assessment and interpretation. This facilitation in surface detection aims to provide medical practitioners with imaging data that can enhance diagnostic capabilities and decision-making efficiency.

The shading component which is configured for executing the step of shading within the rendering method may integrate a function that utilizes specific properties of the transition interval (also: boundary interval) to enhance rendering. The transition interval may be defined as interval or segment along the ray, which is defined by the lower and upper boundary threshold. Enhancements achieved through shading aim to support the portrayal of structures within medical volume data, aiding in diagnostic processes.

One advantageous property to consider is the distance between the lower and upper boundary positions within the transition interval. This parameter can assess spatial variations that may influence shading intensity and coloration. By considering distance, the shading function potentially facilitates depth perception and the delineation of transitions within the volume data, which can be helpful for understanding layered anatomical structures.

An additional property is the magnitudes of gradients at either or both the lower and upper boundary positions or within the transition interval. Gradient magnitude can highlight variations in data intensity, indicating potential surface or material changes. Incorporating gradient information into shading transforms these variations into visual cues, aiming to enhance contrasts in the rendering and make recognizable medical features.

Moreover, the method may incorporate statistical characteristics of sampling values obtained at one or both boundary positions or within the transition interval. Statistical characteristics such as mean, variance, and skewness can contribute to achieving informative shading effects. They serve to ensure that the depiction of surfaces corresponds with the volumetric properties, reflecting the variability and biological features present in the data.

Utilizing these properties in the shading function serves to emphasize intricate details within the medical visualization, improving clarity and relevance for diagnostic analysis. The integration of such shading methodologies may direct attention to areas within the data, guiding practitioner focus and facilitating interpretation of medical conditions.

In the step of shading, derived properties can be calculated at the surface intersection point found in the previous step or in its "proximity". Proximity means the local neighbourhood around the determined surface intersection

As mentioned above, different types of 3D imaging devices may be used. For example, it is possible to use conventional 3D imaging devices or to use a multi-spectral imaging device. Thus, alternatively or cumulatively to the aspects mentioned above, the 3D imaging device may be a multi-spectral imaging device and the provided volumetric dataset may be a multi-spectral dataset and wherein a set of representations is provided as reconstruction result and wherein the set of representations coincide spatially. In the following, this use case or the set of related embodiments is also called multi-spectral case.

In general, multi-spectral 3D imaging, in particular multi-spectral computed tomography (also known as dual energy CT or multi-energy CT or photon counting) uses different X-ray energies to analyse tissue and material compositions more precisely. Multi-spectral imaging 3D imaging may not only be applied for CT or x-ray, but also for other modalities, like MRI, PET, or others. Generally, multi-spectral 3D imaging is an advanced imaging technique. For CT devices, it captures image data at multiple wavelengths across the electromagnetic spectrum. For other devices, other means accomplish this task.

In general, multi-spectral imaging in medicine utilises the acquisition of images in different spectral ranges to obtain more information about the tissue than with traditional imaging techniques. By analysing the spectral differences, differences in the composition, function and/or disease state of the tissue can be better depicted.

The provided volumetric raw dataset, being multi-spectral in nature, includes multiple layers of information that can be advantageous for comprehensive medical analysis. These datasets might capture varying responses from biological tissues or anatomical features, potentially revealing details not visible in standard imaging scenarios. As part of the method, the reconstruction result includes a set of representations-all deriving from the multi-spectral data-capable of spatial alignment.

This spatial coherence among representations is beneficial as it facilitates alignment across different spectral channels and supports the merging of diverse information into a singular visualization. These aligned representations can be synthesized using computational methods that aim to retain the compositional integrity of data from each channel. Spatial alignment can assist in creating visualizations that simultaneously present insights from multiple spectral dimensions, thereby enhancing the practitioner's understanding of volumetric properties or conditions.

The use of multi-spectral imaging and reconstruction may foster the creation of detailed and multispectral-enhanced visualizations, providing depth beyond conventional imaging techniques. This methodology offers medical professionals accurate and spectrally detailed renditions, which may be advantageous for diagnosing conditions that exhibit distinct behaviors across varied wavelengths.

In the multi-spectral case, in the step of sampling: a first mask may be used to determine which representation in the set of representations is used to retrieve the sample value for a given sample position.

This first mask serves as a guide to navigate through multi-spectral data, highlighting a useful representation within the dataset for each sample position. The application of such a mask can assist in engaging accurate and context-specific data for sampling, thereby addressing the complexities present within each spectral channel.

Including a mask in sampling processes can be advantageous, as it potentially offers a structured means of accessing data points across a complex multi-layered dataset. It can enhance the quality and relevance of sampled values, supporting visualizations that reflect precise and insightful characteristics of the underlying volume data. This method may support the generation of informative medical visualizations, capturing nuances and details across different spectral dimensions, and thereby aiding in comprehensive analysis and decision-making in medical diagnostics.

Alternatively or cumulatively, in the multi-spectral case, in the step of sampling: a second mask may be used to determine which set of representations contributes to individual data values when calculating the interpolated sample value.

Thus, In the sampling step, this second mask can be implemented to improve the handling of multi-spectral datasets, assisting in the accuracy of calculating interpolated sample values from the available set of representations. The second mask serves as a useful tool designed to guide the contributions from each representation set to the calculation of individual data values.

By applying a second mask, the method enables the interpolation process by attributing suitable weight and value to representations based on their relevance to specific sampling points. This may involve examining the spectral qualities or positional features of each representation, allowing the interpolated sample value to reflect a comprehensive synthesis of the varied, contributing data layers. This mask assists in determining which channels or spectral representations optimally inform the sample value being calculated.

Implementing the second mask can enhance the fidelity and precision of data handling, permitting rendered visualizations to be appropriately representative of the complex interactions present in multi-spectral datasets. This comprehensive approach can improve the reliability and interpretive value of the resulting medical visualizations, assisting practitioners by providing detailed and nuanced imagery beneficial for diagnostic assessments and careful analysis.

A mask is to be interpreted as data structure for selecting data in another data structure. A mask is configured to select portions of a representation by means of binary voxel encoding for each voxel. A suitable data structure might be a label volume consisting of a suitable number of bits to encode all combinations of representations required by the rendering algorithm. A mask may for instance refer to a labelled or annotated volume used to isolate, select, or protect specific parts of an image representation during processing, editing, or rendering.

Alternatively or cumulatively, in the multi-spectral case, in the step of sampling: different interpolation algorithms for different sets of representations may be used.

Tailored interpolation processes can accommodate the diverse characteristics inherent to spectral data captured across multiple channels, considering the unique data aspects of different representations.

By employing distinct interpolation algorithms, the method may adapt its approach based on the spectral or spatial features present within each subset of representations. For example, one algorithm could be more suitable for high-frequency data variations in one spectral channel, while another might address smoother, low-frequency trends in a different channel. This custom data handling allows sample values to be calculated accurately and reflectively, supporting the integrity and detail of the underlying dataset during visualization.

Such adaptability in interpolation supports more precise merging of multi-spectral data, encouraging visual outcomes that can indicate intricate details present in medical imaging data. This methodological advancement may contribute to creating informative visualizations that assist medical practitioners in diagnostics, enabling detailed analysis through improved clarity and depth in the medical imagery. Utilizing this strategy can lead to nuanced and diagnostically useful visual outputs, facilitating comprehensive evaluations across varied spectral dimensions.

Alternatively or cumulatively, in the multi-spectral case, in the step of sampling: one representation within the set of representations may be selected to be used to decide which interpolation algorithm is to be used for sampling all representations.

With respect to the selection of the representation:
This representation might be selected based on the frequency characteristics. For example, the representation with the highest frequency components might define the interpolation method.

Alternatively or cumulatively, in a local neighbourhood around the sample position, the representation might be selected based on the local frequency characteristics.

Alternatively or cumulatively, depending on the medical use case, one representation might be most important and be used to define the interpolation method.

With respect to the selection or decision on the interpolation algorithm:
Selection of the interpolation algorithm may be based on the frequency characteristics of the different representations. For example, higher frequencies might be interpolated with a larger interpolation kernel.

Alternatively or cumulatively, pre-configured interpolation based on each representation. This can be based on the medical use case or user configuration.

Alternatively or cumulatively, for path-traced SSD, computationally cheaper interpolation methods might be used after a number of scatter events.

Thus, in the sampling step, a singular representation within the complete set of multi-spectral representations may be selected to guide the choice of interpolation algorithm potentially suitable for processing other datasets present within that set. This approach aligns the overall sampling strategy with characteristics or qualities of the chosen representation, aiming for optimization in handling the diverse data spectra.

Selecting one representation as a reference allows for the interpolation algorithm to adapt to the dataset's spectral attributes, offering cohesive sampling outcomes. The representation chosen for this role may display traits deemed beneficial for rendering, such as its spectral sharpness, spatial attributes, or prominence in depicting medically relevant features.

Employing a unified interpolation algorithm informed by a key representation might enable consistent processing, aligning spectral dimensions to a common standard. This method can facilitate visualizations to potentially be more accessible for interpretation by medical professionals. Through careful selection and application of interpolation algorithms, the approach seeks to provide imagery suitable for diagnostic purposes, potentially supporting medical evaluation with enhanced clarity and depth.

Alternatively or cumulatively, in the multi-spectral case, for rendering multi-spectral medical visualizations an approach may be applied suitable for surface intersection detection, utilizing diverse datasets obtained from various spectral representations. The method can include steps to enhance the rendering quality and precision of visualizations.

Starting with a search process, the method examines the lower boundary of the transition interval within a specified first set of representations while concurrently investigating the upper boundary within a different second set of representations. This differentiation between datasets supports specificity and contextual relevance in boundary exploration, aiding in capturing variations and transitions across multi-spectral data channels.

The method can also involve determining representation-specific surface intersection positions for each dataset independently. This allows surface intersections to be distinguished according to the unique qualities of each representation's data, potentially providing a set of prospective intersection points. The detection of a final, unified surface intersection point may be achieved by employing a statistical function to evaluate these individual positions, aiming to obtain a statistically coherent representation intersection that accounts for variations captured by individual spectral channels.

After finalizing ray generation and sampling, a ray segment may be selected for continued surface intersection detection for particular representations. This selection corresponds with the interval defined by the lower and upper boundary positions derived from another representation set. By aligning ray segments using bounds identified from various spectral channels, the integrity of surface data across multi-spectral imagery can be preserved.

Integrating these procedures into the rendering methodology supports dynamic, context-sensitive intersection detection, enhancing accuracy in visual portrayals. This approach assists in illustrating complex anatomical structures, promoting diagnostic practices through imagery that utilizes data captured through multi-spectral imaging.

The statistical function may comprise at least one of the following functions: an average function, a minimum function, a maximum function, a median function, a standard deviation function, and/or a variance function.

Alternatively or cumulatively, in the multi-spectral case, the shading step within the method may comprise deriving shading parameters from properties of multi-spectral representations, which can refine the portrayal of medical data for improved diagnostic visibility. Several processes may be considered to guide the derivation of shading parameters.

Initially, multiple surface normals, tangents, and bitangents may be calculated across the set of representations. These vectors describe the geometric attributes of surfaces at various points within the dataset, detailing orientation and direction that can potentially influence shading effects. A combination of these vectors is usable to establish guidable vectors, aiding the shading function to foster surface realism and illumination accuracy.

Furthermore, multiple curvature values might be calculated for each representation, with their combination shaping adaptable curvature parameters. Curvature provides insights into surface smoothness and bends, possibly affecting how light interacts within the rendered visualization. Combining curvature values across spectral datasets in this manner helps the resultant shading reflect surface dynamics accurately.

Material properties such as reflectance, opacity, and texture variation are definable based on statistical functions of spectral properties captured within the datasets. Evaluating these properties by applying statistical analyses may reveal consistent material features across channels. This calculation assists the shading function in mimicking medical tissue interaction, potentially improving representation fidelity and readability for medical professionals.

Utilizing these steps to derive shading parameters supports rendered visuals in maintaining accurate medical detail, potentially highlighting diagnostically relevant features. The method aligns shading techniques with nuanced multi-spectral data properties, potentially assisting medical practitioners in their diagnostic interpretations and evaluations.

The statistical function may be an average function and/or another function as mentioned before in the context of the step of surface intersection detection.

The final vectors may relate to the final normal, tangents and/or bitangents.

The method may alternatively or in addition, include a global illumination rendering algorithm designed to enhance the visualization of multi-spectral data, featuring a variant of path-traced Shaded Surface Display (SSD). This algorithm selection facilitates comprehensive light simulation techniques, which can contribute to achieving realism and depth in medical imagery by imitating complex light interactions within the volumetric dataset.

During the path or ray generation step, the method offers options for initiating visualization using randomized rays. This randomized approach can offer dynamic and varied sampling across the dataset, potentially highlighting minute features within multi-spectral data that more fixed methods might miss. The use of randomized ray starts provides flexibility in capturing data interactions and supports visually diverse renderings with varied lighting models.

For establishing ray paths, the method offers options for using individual randomized ray starts for each representation within the multi-spectral set or employing a common randomized ray start for all representations. The individual approach allows each spectral channel to have its unique pathway, adapting to inherent channel-specific attributes. This may facilitate nuanced variations to be observed across independent representations, offering detailed insights.

Alternatively, a common randomized ray start across all representations supports synchronized sampling and coherent data integration, where unified ray starts facilitate a collective rendering strategy with streamlined calculations. This approach can promote cohesion and unified interaction within multi-spectral data, enhancing the amalgamation of visual information in a single rendering output.

These rendering techniques are capable of thorough depictions of medical features across a dataset, utilizing each representation's spectral qualities. By providing diversified or unified ray start options, the method provides flexibility according to either individual channel insights or synchronized depiction emphasis. The outcome can assist medical practitioners by delivering detailed and spectrally inclusive imagery that may be well-suited for diagnostic evaluations.

The global illumination rendering algorithm may alternatively use a Metropolis Light Transport, Radiosity, and/or Photon Mapping.

Alternatively or cumulatively, the method can employ a global illumination rendering algorithm, such as a path-traced Shaded Surface Display, to potentially optimize visualization processes for multi-spectral medical datasets. In the surface intersection detection phase, strategic selection of one suitable representation set for each path segment may improve the rendering's specificity and contextual relevance.

Path segments can be distinguishable through factors that may guide the selection of the appropriate representation set. These factors might include inter alia at least one (or a combination of) of the following aspects:
- Type of Path Segment: Path segments may align with particular data characteristics or dimensional features within multi-spectral imagery, suggesting tailored representation alignment (for example primary ray, scattered ray, shadow ray);
- Type of Scattering Event: Path segments originating from certain scattering events could benefit from distinct representations that offer insights into the dataset's behavior under various scattering conditions, e.g. from which type of BxDF;
- Position Signal: Assessing whether a path segment scattered closer to either the lower or upper boundary position might assist in matching representation sets with regions of interest within those defined boundaries;
- Current Path Length: The ongoing length of a path segment may inform representation choice, considering aspects like depth penetration or cumulative scattering effects within the dataset, and/or
- Configuration: Pre-set or defined configurations can support representation set selection based on anticipated medical visualization objectives or potential requirements.

This focused decision-making model may enhance the quality and relevance of intersection detection. By selecting suitable representation sets, the rendering can achieve improved accuracy in portraying surfaces and transitions unique to the multi-spectral medical data. This provides diagnostic processes where practitioners receive clearer and contextually informed visualizations tailored to specific imaging circumstances, thereby aiding potential medical evaluations and considerations.

A configuration may be a user configuration, detected e.g. as parameter input on a user interface and/or as input dataset entered by means of an application. The configuration may be based on heuristic and/or analytical data.

Alternatively or cumulatively, the rendering algorithm is a global illumination rendering algorithm, in particular a path-traced SSD algorithm, and the step of surface intersection detection (S33) comprises simulating translucent materials by defining and traversing a participating medium inside the object defined by the detected surfaces.

Alternatively or cumulatively, the surface intersection detection within the rendering method can connect to simulation techniques for translucent materials, which may offer enhancement options in realism and detail in medical visualizations. By employing a global illumination rendering algorithm, such as a path-traced Shaded Surface Display, the visualization method may define a participating medium.

This participating medium can refer to material characteristics simulated inside objects delineated by the detected surfaces. Translucent material simulation might depict light interactions that occur as rays traverse or are absorbed by the medium, supporting the representation of semi-transparent anatomical structures within the volume data.

By traversing the participating medium, nuanced visualization may be encouraged, where intricate details affected by translucency could be emphasized. This approach may suit a sophisticated light simulative method, which can portray volumes with layered complexity, addressing features such as material density variations that provide a diagnostic option.

The integration of translucent material simulation within surface intersection detection is intended to advance visualization. This simulation aims to represent medical features that might exhibit varying translucency under real-world conditions, creating imagery that medical professionals might find beneficial for observational and evaluative purposes in diagnostics. By considering light diffusion, absorption, and scattering as it interacts within the participating medium, the rendering method intends to offer visuals that may be filled with lifelike clarity and layered depth, with optimization possibilities for medical analysis.

In another aspect the invention relates to a rendering machine for rendering medical visualizations of 3D or volume data, acquired by an 3D imaging device. The rendering machine can be structured into several components to perform multiple rendering steps or integrate features compatible with the described methods. In particular, the rendering machine is configured to perform any of the steps of the rendering algorithm/method as described above.

The rendering machine may comprise a receiving interface as one component for receiving or providing a reconstruction result, wherein the reconstruction result comprises a 3D volume. This receiving interface enables efficient transfer and handling of volumetric data, preparing the machine to process information that supports rendering visualizations.

The rendering machine may further comprise another component, namely a computing unit configured for rendering medical visualizations, acquired by an 3D imaging device.

The computing unit may be configured for executing a rendering algorithm for calculating a visualization for the received or provided reconstruction result, and wherein executing the rendering algorithm comprises the steps of ray generation, sampling for determining sampling values, surface intersection detection and shading.

In the step of surface intersection detection for calculating at least one surface intersection point, the computing unit may be configured for calculating, a first surface intersection point for a lower boundary of a transition interval and calculating a second surface intersection point for an upper boundary of the transition interval and applying a detection function on the calculated first and second surface intersection point for determining the surface intersection point and/or rendering two surfaces, namely at the lower and the upper boundary and composing two surface colours of the rendered surfaces to calculate a final colour for the ray.

Alternatively or in addition, in the step of shading, the computing unit may be configured for using the determined sampling values which have been determined during the step of sampling (in particular instead of the material colour) and/or determining a surface colour by using a shading function, which is based on properties of the transition interval and/or determining material properties and/or parameters of a BxDF function, based on the properties of the transition interval.

The rendering machine may further comprise a display unit as a further component which is configured for displaying the rendered visualization. Alternatively, the rendering machine may comprise an interface to such a display unit, so that the display unit may be external to the rendering machine. So, for example, the rendered images may be transferred to mobile devices via radio or wireless transmission. Presenting detailed and accurate volume data visualizations, the rendering machine assists medical practitioners in effectively interpreting medical conditions. Integrating advanced rendering technology enhances the quality and effectiveness of medical imagery for diagnostic applications, providing precise information to aid medical analysis and decision-making.

In another aspect, the invention refers to a medical image processing system for rendering medical visualizations, acquired by an 3D imaging device, with the 3D imaging device, a reconstruction computer (reconstruction engine) configured for providing a reconstruction result, wherein the reconstruction result comprises a 3D volume and a rendering machine as described above.

The medical image processing system may comprise a display unit.

In state-of-the-art systems, the information used by the rendering algorithm during surface intersection detection and shading from the individual reconstructed volumes is not utilized to its full potential. Moreover, it is currently not possible to combine the values and characteristics of multiple reconstructed volumes during the actual rendering process. These disadvantageous aspects are both overcome by the solution provided herein. In particular, the information from each of the individual reconstructed volumes is used during rendering and in the rendering algorithm and/or values and characteristics of multiple reconstructed volumes are considered in the rendering algorithm.

As an advantage, it is possible to take multiple reconstruction results into account to produce a single rendered image. For this, the respective algorithms themselves need to incorporate the multiple reconstruction results.

In addition to multi-spectral opportunities, the SSD algorithm can also be extended for scalar data to convey more information about the surface characteristics at the intersection point.

As to a further aspect, a computer program product is provided comprising program elements which induce a computing device to carry out the steps of the method for providing a rendered visualization as result according to the method aspect, when the program elements are loaded into a memory of the computing device.

As to a still further aspect, a computer-readable medium is provided on which program elements are stored that can be read and executed by a computing device, in order to perform steps of the method for providing a rendered visualization according to the method aspect, when the program elements are executed by the computing device.

The properties, features and advantages of this invention described above, as well as the manner they are achieved, become clearer and more understandable in the light of the following description and embodiments, which will be described in more detail in the context of the drawings.

This following description does not limit the invention on the contained embodiments. Same components or parts can be labelled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### Brief description of the drawings

- Fig. 1: is a flow chart of a method for rendering medical visualizations of volume data, acquired by an 3D imaging device in different embodiments of the present invention;
- Fig. 2: is a flow chart of main steps of a rendering algorithm;
- Fig. 3: is a more detailed flow chart for the step of surface intersection detection of the rendering algorithm according to a preferred embodiment of the present invention;
- Fig. 4: is a more detailed flow chart for the step of shading of the rendering algorithm according to a preferred embodiment of the present invention;
- Fig. 5: schematically illustrates a computing architecture for a rendering machine with related entities according to a preferred embodiment of the present invention;
- Fig. 6: shows a more detailed schematical representation of a computing architecture in an alternative embodiment as the one of Fig. 5; and
- Fig. 7: represents a general computing architecture for rendering medical visualizations of 3D data.

Any reference signs in the claims should not be construed as limiting the scope.

In this disclosure, it is suggested to incorporate different representations into the actual rendering algorithms themselves. In the following, various opportunities for using different sets of representations for each step of both the SSD and Path-traced SSD algorithms are shown.

In addition, novel methods also for the scalar case are disclosed that enable conveying additional properties of the volume in the proximity of the surface point.

For Path-traced SSD algorithms, all options which are described for the SSD algorithm do also apply.

Conventional SSD rendering and Path-traced SSD rendering algorithms only uses a single scalar representation of multi-spectral data to generate a final image, whereas in the modified rendering algorithms multiple representations are used to generate or render the final image.

This invention leverages the added information contained in multi-spectral data to be visualized in new ways. This is achieved by incorporating the multi-dimensionality of multi-spectral data directly into the actual rendering techniques themselves before determining the final output values. By these means, the radiologist can gain new insights into the information encoded in the multi-spectral data.

Moreover, this invention proposes numerous new extensions also to the scalar SSD / Path-traced SSD algorithms that allow novel ways to visualize the volume data.

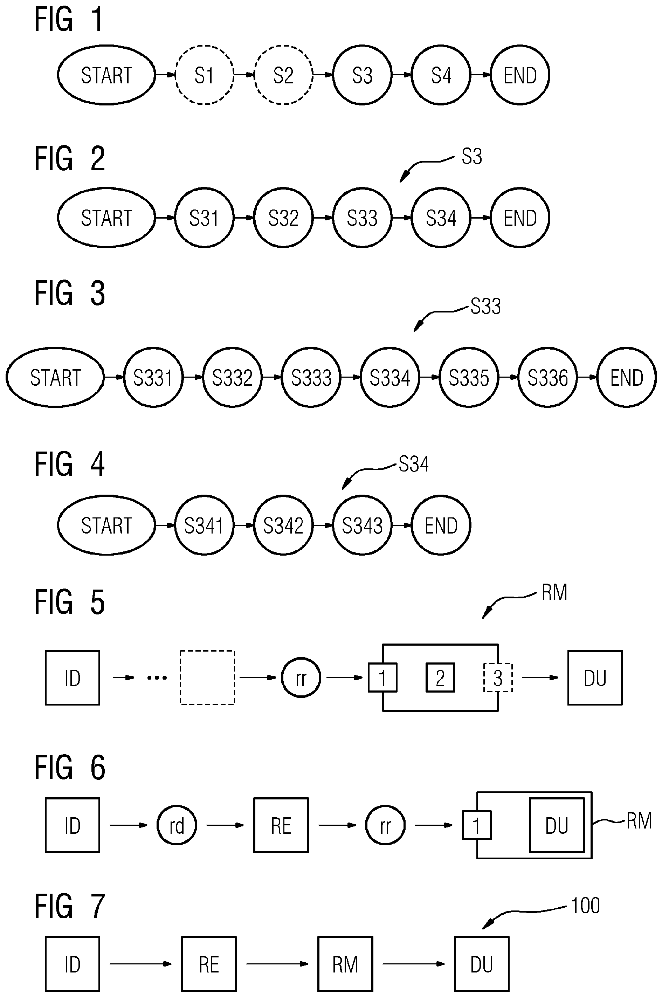

Fig. 1 schematically illustrates an exemplary flowchart for a (in particular computer-implemented) method for rendering medical visualizations of volume data, acquired by an 3D imaging device ID.

The first two steps are optional and are depicted in Fig. 1 with dotted lines.

In a first step S1, the rendering method may comprise executing a 3D image acquisition at the 3D imaging device (ID) for providing a raw dataset rd.

In a second step S2, also optional, the rendering method may comprise executing a reconstruction. In this step, a reconstruction result rr may be provided with a 3D volume, reconstructed from the provided raw dataset rd. The reconstruction result rr may be comprise or refer to a representation of a scalar volume.

Alternatively, the method may access for example a memory or storage which is configured for storing the raw dataset rd and/or the reconstruction result rr. In this embodiment, the image acquisition and/or the reconstruction phase may be decoupled from the rendering phase. The rendering phase does, thus, not necessarily require the image acquisition and the reconstruction to be executed in the same time phase or instantaneously. In particular, at least one of these steps (image acquisition or reconstruction) may be executed earlier or in an earlier time phase and the respective resulting datasets are stored in a storage. The modified rendering algorithm accesses the storage.

A key aspect of the present invention is to be seen in that the rendering algorithm is modified. In particular, the steps of surface intersection detection and shading are modified.

A thus modified rendering algorithm (in the following only mentioned as rendering algorithm) is executed in step S3. It serves for calculating a visualization for the previously provided reconstruction result rr. The reconstruction result rr comprises a 3D volume. Details of the modified rendering algorithm will be explained below in Fig. 2, 3 and 4.

In step 4, the rendered visualization is displayed on a display unit, like a monitor or a computer screen. After this, the method may end.

Fig. 2 represents the step S3 of the rendering algorithm in more detail. In step S31 a ray or path generation process may be executed. In step S32 sampling may be executed. This refers to determining sampling values to be used in later steps of the rendering algorithm. In step S33 a surface intersection detection may be executed. Finally, in step S34 a shading is performed. These steps are also applied in the conventional rendering algorithms.

However, as can be seen in Fig. 3 and 4, in particular the second and third step are modified or adapted to consider more information from multiple reconstruction results.

Fig. 3 shows possible adaptions of the step of surface intersection detection S33.

In step S331a first surface intersection point for a lower boundary of a transition interval is calculated. In step S332 a second surface intersection point for an upper boundary of the transition interval is calculated. In step S333 a detection function is applied on the calculated first and second surface intersection point for determining the (then final) surface intersection point. The detection function uses these two values (of the first and second surface intersection point) for detecting or determining the actual surface intersection position or point.

Alternatively or in addition, in step S334 one or two surfaces, namely at the lower and the upper boundary may be rendered. Alternatively or in addition in step S335 two surface colours of the rendered surfaces are composed to calculate a final colour for the ray in step S336.

In general, instead of searching only one transition into the threshold range along the ray, the adapted surface intersection detection or detection function may search both the lower and upper boundary transition and use these two values for detecting the actual surface intersection position in a combination of:
- Either the lower or upper boundary position, depending on the sampled value or derived properties (e.g. gradient magnitude, curvature) at the respective positions.
- A weighted average of the lower and upper boundary position, with the weighting factor being defined by the application, the user or derived from the properties of the volume in total or in a local proximity around the boundary positions.
- The position within the boundary interval with the largest gradient magnitude.
- The position within the boundary interval where the surface is most precisely defined, with respect to either:
- Highest continuity in the sample values of the neighborhood
- Highest continuity of the gradients of the neighborhood
- Instead of determining and rendering only a single surface intersection position, render two surfaces, one at the lower and one at the upper boundary position. Compose the two surface colors to calculate the final color for the ray.

For multi-spectral data, the following extensions to the scalar SSD algorithm are proposed:
- Search the lower boundary in one set of Representations and the upper boundary in another set of Representations, using the respective threshold values.
- Determine the surface intersection position for each Representation separately, then determine the final surface position based on a combination of these positions, in particular by using a statistical function.
- Limit the ray segment for the surface intersection detection of one set of Representations by the interval defined by the lower and upper boundary positions found from another set of Representations.

Fig. 4 shows possible adaptions of the step of shading S 34. In the step S341 the determined sampling values which have been determined during the step of sampling S32 are used for shading instead of the material colour. Alternatively or in addition, a surface colour is determined by using a shading function S342. The shading function processes or is based on properties of the transition interval. In step S343 material properties and/or parameters of a BxDF function (such as opacity, specularity, roughness, anisotropy, refraction, and/or sub-surface scattering parameters) are determined, based on the properties of the transition interval.

In general, the step of shading may be adapted by using the sampled value instead of the material color.

Alternatively or cumulatively, during shading the surface color may be determined using a function depending on properties of the boundary interval, in particular:
- The distance between the lower and upper boundary position.
- The magnitudes of the gradients at one or both boundary positions or within the boundary interval.
- The statistics of sampling values at one or both boundary positions or within the boundary interval.

Alternatively or cumulatively, during shading the material properties and/or parameters of the BxDF (such as opacity, specularity, roughness, anisotropy, refraction, sub-surface scattering parameters) may be defined based on the characteristics of the boundary interval, as described in the previous method.

For multi-spectral data, the following extensions to the scalar SSD algorithm are proposed:
- Derive shading parameters from properties from a set of Representations:
- Calculate multiple surface normals, tangent and/or bitangent for a set of Representations then use a combination of these as the final vectors.
- Calculate multiple curvature values (principal curvatures, Gaussian, etc.) for a set of Representations then use a combination of these as the final curvature values.
- Define material properties described above based on the statistics of the multiple properties.

Fig. 5 shows a rendering machine RM as a hardware implementation which may be configured for executing the rendering algorithm as described above.

The rendering machine RM comprises a receiving interface 1 for receiving the reconstruction result rr from external devices, in particular from the 3D imaging device ID and/or from a reconstruction engine RE or from another device, e.g., a storage, depicted in Fig. 5 with dotted lines as an alternative. Alternatively or in addition the receiving interface 1 may be configured for providing the (in particular received) reconstruction result rr to the computing unit 2. The reconstruction result rr comprises the 3D volume.

As further component, the rendering machine RM comprises the computing unit 2 which is configured for rendering medical visualizations of the volume data, acquired by the 3D imaging device ID.

The computing 2 unit is configured for executing the (modified) rendering algorithm S3 for calculating a visualization for the received and/or provided reconstruction result rr. When executing the rendering algorithm S3 the steps of ray generation S31, sampling S32 for determining sampling values, surface intersection detection S33 and shading S34 are executed.

The rendering algorithm is modified in that in the step of surface intersection detection S33 for calculating at least one surface intersection point, the computing unit 2 is configured for calculating S331, a first surface intersection point for a lower boundary of a transition interval and calculating S332 a second surface intersection point for an upper boundary of the transition interval and applying a detection function S333 on the calculated first and second surface intersection point for determining the surface intersection point and/or rendering S334 two surfaces, namely at the lower and the upper boundary and composing S335 two surface colours of the rendered surfaces to calculate S336 a final colour for the ray.

Alternatively or in addition the rendering algorithm is modified in that in the step of shading S34, the computing unit 2 is configured for using the determined sampling values which have been determined during the step of sampling S32 in step S341 and/or determining a surface colour by using a shading function S342, which is based on properties of the transition interval and/or determining S343 material properties and/or parameters of a BxDF function, based on the properties of the transition interval.

The rendering machine RM may comprise an internal or external display unit DU which is configured for displaying the rendered visualization. The display unit DU may be part of the rendering machine RM itself or may be connected via a respective output interface 3.

Optionally, and shown in Fig. 5 in dotted lines, the rendering machine RM may comprise an output interface 3, which is configured for providing the rendered visualization, so that the rendered visualization may be provided to an external display unit for display.

Fig. 6 shows the process or visualization pipeline in a schematic illustration. The 3D imaging device ID is configured to acquire a raw dataset rd. The raw dataset may be subject to reconstruction by means of a reconstruction engine RE. The reconstruction engine RE is configured to provide a reconstruction result rr, which in an embodiment, may be provided via the receiving interface 1 to the rendering machine RM, which in the embodiment depicted in Fig. 6 may comprise the display unit DU.

Fig. 7 schematically depicts the structural architecture of a medical image processing system 100 according to an embodiment. The medical image processing system 100 is configured for rendering medical visualizations of volume data, acquired by the 3D imaging device ID. The system comprises the 3D imaging device ID, a reconstruction engine RE configured for providing a reconstruction result rr, which comprises a 3D volume. The system further comprises the rendering machine RM. As mentioned above, the rendering machine RM may comprise or may be connected to a display unit DU for display of the rendered images.

Independent of the grammatical term usage, individuals with male, female or other gender identities are included within the term.

## Claims

1. Computer-implemented method for rendering medical visualizations of volume data, acquired by an 3D imaging device (ID), comprising the following method steps:
- Executing a rendering algorithm (S3) for calculating a visualization for a previously provided reconstruction result (rr), wherein the reconstruction result (rr) comprises a 3D volume and wherein executing the rendering algorithm (S3) comprises the steps of ray generation (S31), sampling (S32) for determining sampling values, surface intersection detection (S33) and shading (S34);
- in the step of surface intersection detection (S33) for calculating at least one surface intersection point: calculating (S331), a first surface intersection point for a lower boundary of a transition interval and calculating (S332) a second surface intersection point for an upper boundary of the transition interval and applying a detection function (S333) on the calculated first and second surface intersection point for determining the surface intersection point and/or rendering (S334) two surfaces, namely at the lower and the upper boundary and composing (S335) two surface colours of the rendered surfaces to calculate (S336) a final colour for the ray; and/or
- in the step of shading (S34): using (S341) the determined sampling values which have been determined during the step of sampling (S32) and/or determining a surface colour by using a shading function (S342), which is based on properties of the transition interval and/or determining (S343) material properties and/or parameters of a BxDF function, based on the properties of the transition interval;
- Displaying (S4) the rendered visualization.

2. Method according to claim 1, wherein the method comprises:
- Executing (S1) a 3D image acquisition at the 3D imaging device (ID) for providing a raw dataset (rd); and/or
- Providing (S2) a reconstruction result (rr) with a 3D volume, reconstructed from the provided raw dataset (rd) as a representation of a scalar volume.

3. Method according to any one of the preceding claims, wherein the rendering algorithm is a shaded surface display algorithm, SSD, or path-traced SSD.

4. Method according to any one of the preceding claims, wherein in the step of surface intersection detection (S33), the detection function (S333) is configured to execute at least one of the following steps:
- Determining the calculated first or second surface intersection point as the detected surface intersection point based on the sampled value or derived properties at the respective position of the first or second surface intersection point;
- Applying a weighted average of the first and second surface intersection point, wherein a weighting factor is definable by an application, a signal on a user interface and/or derived from properties of the volume in total or in a local proximity around the upper and/or lower boundary of the transition interval;
- Determining a position within the transition interval with the largest gradient magnitude as the detected surface intersection point; and/or
- Determining a precision position within the transition interval as the detected surface intersection point, where a surface is most precisely defined, with respect to either:
- Highest continuity in the sample values of the neighbourhood and/or
- Highest continuity of the gradients of the neighbourhood.

5. Method according to any one of the preceding claims, wherein in the step of shading (S34), the shading function (S342) uses at least one of the following properties of the transition interval:
- a distance between the lower and upper boundary position,
- the magnitudes of the gradients at one or both of the lower and upper boundary positions or at positions within the transition interval, and/or
- statistical characteristics of sampling values at one or both boundary positions or at positions within the transition interval.

6. Method according to any one of the preceding claims, wherein the 3D imaging device (ID) is a multi-spectral imaging device and the provided volumetric raw dataset (rd) is a multi-spectral dataset and wherein a set of representations is provided as reconstruction result (rr) and wherein the set of representations coincide spatially.

7. Method according to claim 6, wherein in the step of sampling (S32): a first mask is used to determine which representation in the set of representations is used to retrieve the sample value for a given sample position.

8. Method according to any of claims 6 or 7, wherein in the step of sampling (S32): a second mask is used to determine which set of representations contributes to individual data values when calculating the interpolated sample value.

9. Method according to any one of the claims 6 to 8, wherein in the step of sampling (S32): use different interpolation algorithms for different sets of representations.

10. Method according to any one of the claims 6 to 9 wherein in the step of sampling (S32): one representation within the set of representations is selected to be used to decide which interpolation algorithm is to be used for sampling all representations.

11. Method according to any one of the claims 6 to 10, wherein the step of surface intersection detection (S33) comprises:
- Searching the lower boundary of the transition interval in a first set of representations and searching the upper boundary of the transition interval in a second set of representations, wherein the first and the second set of representations are different, by using values of the lower and upper boundary,
- Determining representation-specific surface intersection positions for each representation separately, and detecting the surface intersection point based on a statistical function thereof;
- After the steps of ray generation (S31) and sampling (S32): Selecting a ray segment for the surface intersection detection of one set of representations by the interval defined by the lower and upper boundary positions found from another set of representations.

12. Method according to any one of the preceding claims 6 to 11, wherein the step of shading (S34) comprises deriving shading parameters from properties from a set of representations by at least one of the following steps:
- Calculating multiple surface normals, tangents and/or bitangents for a set of representations and using a combination thereof as final vectors,
- Calculating multiple curvature values for a set of representations and using a combination thereof as final curvature values, and/or
- Defining material properties based on at least one statistical function of at least one property.

13. Method according to any of the preceding claims 6 to 12, wherein the rendering algorithm (S3) is a global illumination rendering algorithm, in particular a path-traced SSD algorithm and in the step of ray generation (S31): using individual randomized ray starts for each representation in the set of representations or using a common randomized ray start for all representations in the set of representations.

14. Method according to any of the preceding claims 6 to 13, wherein the rendering algorithm (S3) is a global illumination rendering algorithm, in particular a path-traced SSD algorithm and in the step of surface intersection detection: selecting one decisive set of representation per path segment, wherein the step of selecting is based on at least one of:
- A type of the path segment;
- A type of scattering event, from which the path segment was created from;
- A position signal, indicating whether the path segment scattered closer to the lower boundary position or upper boundary position;
- A current path length, and/or
- A configuration.

15. Rendering machine (RM) for rendering medical visualizations for volume data, acquired by an 3D imaging device (ID), wherein the rendering machine (RM) is configured to perform any of the steps of any of the method claims and/or having any of the features of any of the method claims, comprising:
- A receiving interface (1) for receiving or providing a reconstruction result (rr), wherein the reconstruction result (rr) comprises a 3D volume;
- A computing unit (2) configured for rendering medical visualizations of volume data, acquired by the 3D imaging device (ID),
- wherein the computing (2) unit is configured for executing a rendering algorithm (S3) for calculating a visualization for the received or provided reconstruction result, and wherein executing the rendering algorithm (S3) comprises the steps of ray generation (S31), sampling (S32) for determining sampling values, surface intersection detection (S33) and shading (S34);
- wherein in the step of surface intersection detection (S33) for calculating at least one surface intersection point, the computing unit (2) is configured for calculating (S331), a first surface intersection point for a lower boundary of a transition interval and calculating (S332) a second surface intersection point for an upper boundary of the transition interval and applying a detection function (S333) on the calculated first and second surface intersection point for determining the surface intersection point and/or rendering (S334) two surfaces, namely at the lower and the upper boundary and composing (S335) two surface colours of the rendered surfaces to calculate (S336) a final colour for the ray; and/or
- wherein in the step of shading (S34), the computing unit (2) is configured for using (S341) the determined sampling values which have been determined during the step of sampling (S32) and/or determining a surface colour by using a shading function (S342), which is based on properties of the transition interval and/or determining (S343) material properties and/or parameters of a BxDF function, based on the properties of the transition interval;
- A display unit (DU) or an interface to the display unit (DU), which is configured for displaying the rendered visualization.
